# EUROPEAN PATENT SPECIFICATION

(11) **EP 1 871 239 B1**
(45) Date of publication and mention of the grant of the patent: **13.08.2014**
(21) Application number: 06824698.2
(22) Date of filing: 12.04.2006
(51) Int. Cl.: A61B 17/04

(54) **SUTURE FIXATION DEVICE FOR SURGICAL REPAIR**
NAHTFIXIERUNGSVORRICHTUNG FÜR CHIRURGISCHE REPARATUREN
DISPOSITIF DE FIXATION DE SUTURE POUR UNE REPARATION CHIRURGICALE

(30) Priority: 20.04.2005 US 110419
(43) Date of publication of application: 02.01.2008
(73) Proprietor: Arthroscopic Innovations LLC, Weymouth, MA 02188 (US)
(72) Inventor: CERUNDOLO, Daniel, Weymouth, MA 02188 (US)
(74) Representative: Grey, Ian Michael
(86) International application number: PCT/US2006/013929
(87) International publication number: WO 2007/024282

(56) References cited:
- EP-A2- 0 755 656
- EP-A2- 1 013 229
- US-A1- 2004 204 724

## Description

This invention relates to a suture fixation device for surgical repair.

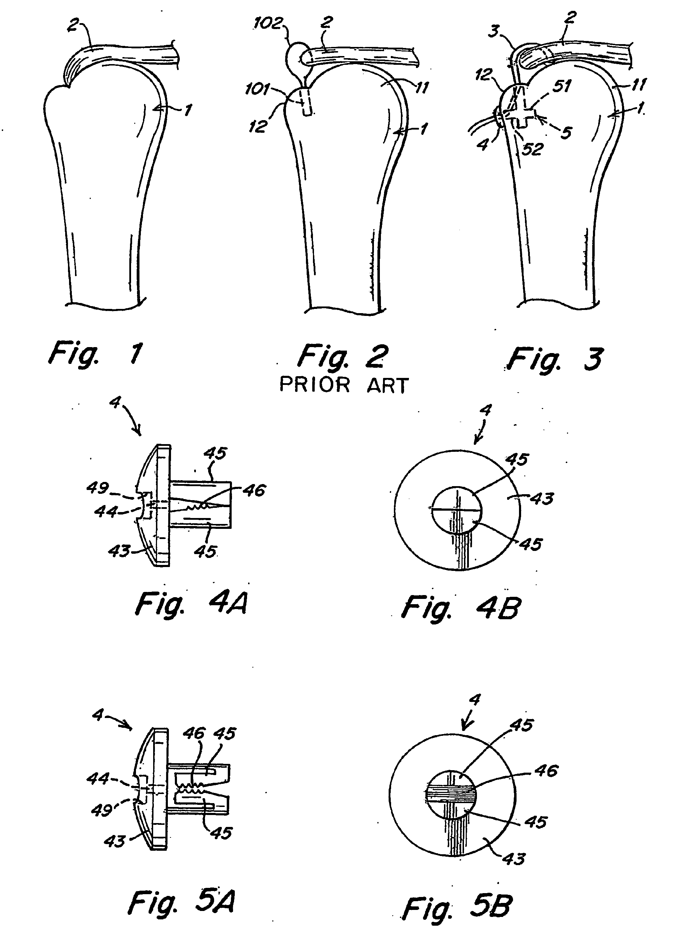

Suture anchors and other suture fixation devices are often used for surgical repair, such as when attempting to secure one body portion relative to another or relative to a surgical implant or other device. For example, tendon damage frequently requires surgery for repair, e.g., to reattach a torn or separated tendon to the bone to which the tendon would normally be attached. Shoulder rotator cuff injuries typically involve damage to the rotator cuff tendon such that the tendon, or at least a portion thereof, requires reattachment to the humerus. Figure 1 shows a schematic diagram of a humerus 1 and a portion of a rotator cuff tendon 2 that is normally attached to the head of the humerus. In one type of damage to the rotator cuff, the tendon 2 may detach or be partially torn from the humerus 1, such as that shown schematically in Figure 2. Such damage may be repaired by reattaching the rotator cuff tendon to the humerus 1 by a suture or other fixation so that the body's normal healing processes can naturally effect reattachment of the tendon to the bone. One repair technique for reattaching the rotator cuff 2 to the humerus 1 involves fixing an anchor 101 at a margin between the articulating portion 11 of the humerus 1 and the humerus' greater tuberosity 12. A suture 102 is secured to the rotator cuff 2 and the anchor 101, and the suture 102 is tensioned so that the rotator cuff 2 is held in place close to the humerus 1. Thereafter, the body may reestablish the proper attachment of the rotator cuff 2 to the humerus 1.

It is known from EP 1013229 to provide a suture fixation device according to the preamble of claim 1.

A suture fixation device according to the present invention is characterised by the features recited in the characterising portion of claim 1.

Preferred features of the suture fixation device are defined in the dependent claims.

Various aspects of the invention are described with reference to illustrative embodiments, wherein like numerals reference like elements, and wherein:
FIGURE 1 is a schematic diagram of a head of a humerus and attached rotator cuff tendon;
FIGURE 2 shows a prior art technique for repairing a rotator cuff injury;
FIGURE 3 is a schematic diagram of a tissue repair arrangement in accordance with an aspect of the invention;
FIGURES 4A-B and 5A-B show side and rear views, respectively, of illustrative embodiments of suture fixation devices in accordance with the invention;
FIGURE 6 shows a suture fixation device engagement tool in engagement with a suture fixation device in accordance with the invention;
FIGURE 7 shows the placement of a suture fixation device relative to the bone in accordance with the invention;
FIGURE 8 shows the engagement of a suture with a suture fixation device in accordance with the invention;
FIGURES 9-11 show the use of a needle for placing a suture in a tissue in accordance with the invention;
FIGURE 12 shows a needle in engagement with a cannula in accordance with an aspect of the invention;
FIGURE 13 shows an illustrative arrangement for engaging a needle with a cannula in one embodiment;
FIGURE 14 shows an illustrative arrangement for engaging a sleeve and needle assembly with a cannula in accordance with another embodiment;
FIGURE 15 shows a guide apparatus used in forming a passageway in accordance with the invention;
FIGURE 16 shows the use of a guide apparatus for passing a suture or other element through a transosseous passageway in accordance with the invention; and FIGURE 17 shows a technique for passing a suture placed in a tissue through a passageway.

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting.

Various aspects of the invention are described below with reference to specific embodiments. For example, aspects of the invention are described in the context of performing a rotator cuff repair. However, it should be understood that aspects of the invention are not necessarily restricted to rotator cuff repair techniques, or even to surgical techniques performed on a shoulder. Rather, various aspects of the invention may be used in any suitable surgical procedure. In addition, various aspects of the invention may be used alone, and/or in combination with any other aspects of the invention.

A method for performing a surgical procedure may include providing a passageway in a body portion where the passageway extends from a first opening in the body portion, e.g., a bone, to a second opening in the body portion. A suture may be placed in or otherwise secured to a material, e.g., a tissue, prosthetic, surgical implant, etc., to be secured relative to the body portion and two ends of the suture extending away from the material may be positioned in the first opening and extend into the passageway. The material may be secured relative to the body portion by securing the two ends of the suture in the passageway relative to the body portion near the second opening. For example, a rotator cuff tendon may be secured to a humerus by a suture that is placed in the tendon and has two ends that extend through a passageway in the humerus having one opening near the rotator cuff and a second opening positioned away from the rotator cuff, such as at a lateral side of the humerus. The two ends of the suture may be positioned in the passageway and secured at or near the second opening at the lateral side of the humerus. In one embodiment, a suture fixation device may be positioned near the second opening to help secure the two suture ends.

The suture fixation device is used to help secure a suture relative to a passageway in bone and is arranged so as to secure the suture relative to the bone at an opening into the bone by having a portion of the suture fixation device positioned outside of and adjacent the opening in contact with portions of cortical bone. By securing the suture (and potentially a tissue or other material engaged with the suture) relative to the bone by contact of the suture fixation device with cortical bone, the suture may be more securely fixed as compared to devices that engage with softer cancellous bone.

The suture fixation device includes a body having an inner end and an outer end and a pathway extending between the inner and outer ends. The inner end is arranged to be positioned in a hole in a body portion, such as bone. The body includes a restriction in the pathway that relatively freely permits movement of a knotless suture through the pathway in a first direction and inhibits movement of the knotless suture through the pathway in a second direction opposite the first direction.

Various aspects of the invention may be used in an open surgical procedure or in a closed procedure, such as an arthroscopic procedure. Also, various aspects of the invention may be used in any suitable surgical or other procedure involving any suitable body portions, such as bone, muscle, skin, vascular structures, digestive structures or other tissue, implants, mesh, or other medical devices, etc.

Figure 3 shows a schematic diagram of a surgical repair in accordance with aspects of the invention. As discussed above, although aspects of the invention are described with reference to a rotator cuff repair for ease of reference and understanding, aspects of the invention may be used in any surgical or other procedure, and may involve any suitable body portions, such as bone, muscle, other tissue or combinations thereof, medical implants or other devices, etc. Thus, aspects of the invention are in no way limited to the specific embodiments and examples described herein.

In this illustrative embodiment, a rotator cuff tendon 2 is secured by a suture 3 relative to a humerus 1. The suture 3 is placed in the tendon 2, for example, using a mattress stitch or other arrangement, and is passed through a passageway 5 formed through the humerus 1. In this embodiment, the passageway 5 is formed by first and second intersecting holes. A first hole 51 is formed vertically as shown in Figure 3 from a first opening at or near a margin between the articulating surface 11 and the greater tuberosity 12 of the humerus 1. The second hole 52 is formed horizontally as shown in Figure 3 from a lateral position on the humerus 1. The suture 3 is secured at the second opening 54 of the second hole using a suture fixation device 4 that is positioned adjacent the second opening 54. Although in this embodiment the first and second holes 51 and 52 are arranged at approximately right angles, the first and second holes may be arranged at any suitable angle and may be colinear (i.e., at a 180 degree angle relative to each other). Alternately, the passageway 5 may be formed by a single, straight hole.

A wire, other material or the suture 3 may be manipulated in the passageway 5 so as to cut through or crush the relatively soft cancellous bone of the humerus in the passageway 5 so that the suture follows a relatively straight path between the first and second openings into the first and second holes 51 and 52. The relatively straight pathway may be formed by a "flossing" operation, such as by using a wire that is passed through the passageway 5 and is manipulated, e.g., tensioned and reciprocally drawn between the first and second openings, so as to cut through or crush the cancellous bone, thereby forming a relatively straight path for the suture 3.

The suture fixation device 4 is arranged so as to contact cortical bone near an opening of a passageway in which the suture is positioned. Such an arrangement may provide for more secure fixation of the suture as compared to suture fixation devices that engage mainly or exclusively with cancellous bone, which is generally "softer" than cortical bone. For example, the suture fixation device 4 includes a flange portion that contacts the bone portion around the hole with which the suture fixation device 4 is associated. Although embodiments of a suture fixation device herein are described as cooperating with a passageway in bone, it should be understood that the passageway may be formed in any suitable body portion in accordance with various aspects of the invention.

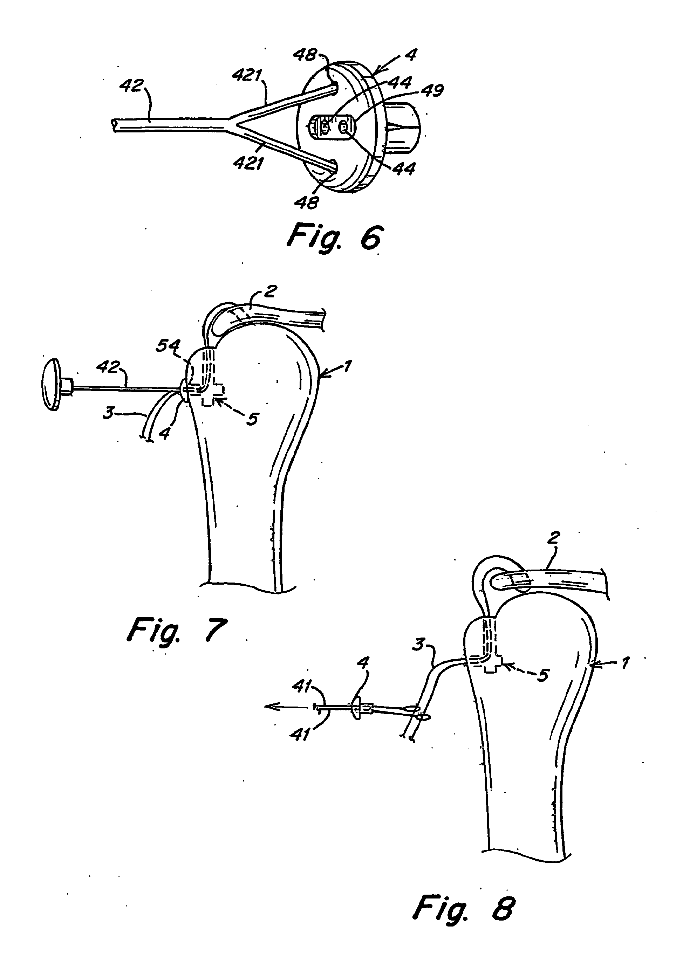

Figures 4A-B and 5A-B show side and rear views of illustrative embodiments of suture fixation devices 4 in accordance with the invention. Figure 6 shows a perspective view of an outer end of the suture fixation devices 4. The suture fixation devices 4 include a restriction in a pathway through the suture fixation device 4 so that suture or other material passing through the pathway is relatively freely moved in one direction through the pathway, but movement of the suture or other material in the other direction in the pathway is resisted. For example, movement of a suture though the suture fixation devices shown in Figures 4A and 5A in a direction to the left may be freely allowed, while movement of the suture toward the right may be resisted. In the embodiment of Figure 3 above, the restriction may aid in maintaining tension on the suture 3, e.g., while a knot is formed in the suture 3. For example, the suture 3 may be pulled from the second hole 52 through the suture fixation device 4 until the rotator cuff or other material is appropriately tensioned. Thereafter, the suture 3 may be temporarily released, e.g., in preparation for forming a knot, but movement of the suture back through the suture fixation device 4 may be resisted so that tension is maintained on the rotator cuff or other material, which may remain in place until the suture knot is tied or the suture is otherwise secured. The restriction in the suture fixation device 4 provides for knotless fixation of the suture. Alternately, knotless fixation of the suture may be provided by other features, such as an interference pin, locking cap, etc.

In Figure 4, the suture fixation device includes an outer end having a flange portion 43 that is sized and arranged to contact the cortical bone adjacent the opening in the passageway at which the suture fixation device 4 is positioned, e.g., the second opening 54. One or more pathways 44 may be formed through the suture fixation device 4, such as by a hole or holes formed through the flange 43 (see Figure 6). Instead of having multiple holes, the pathway 44 may include a single slot arranged to receive one or more sutures. A recess 49 may be provided in the flange portion 43 to receive one or more knots, if formed with the suture(s) in the pathway 44. A pair of duck bill members 45 at an inner end of the suture fixation device 4 extend rearwardly from the flange 43 and are arranged as to be positionable in the second hole 52. A groove between the duck bill members 45 extends across the inner end of the suture fixation device 4 so that the members 45 may move independently of each other. In this embodiment, the groove separating the duck bill structures 45 extends from the flange portion 43 to the innermost end of the device 4 so that the structures 45 are pivotable at a point near the flange portion 43. The duckbill members 45 are resiliently biased toward each other so as to resist the passage of suture or other material through the pathway 44. One or both of the duck bill structures 45 may include serrations 46 or other features that may aid in engaging a suture or other material.

The Figure 5 embodiment similarly includes a flange 43 and one or more pathways 44. Duck bill structures 45 are also provided. However, in this embodiment rather than being hinged at a point near respective connection points with the flange 43, the duck bill portions 45 are hinged at a point positioned away from the flange 43 toward the inner end. Providing the effective hinge points for the duck bill structures 45 in this manner may provide improved engagement of the duck bill structures 45 with a suture or other material when the suture is urged to move from the outer end toward the inner end through the pathway 44. That is, if the suture is pulled to move toward the inner end, serrations 46 or other features may engage with the suture and increased force on the suture will cause an increased force urging the duck bill structures 45 to move toward each other and further squeeze the suture. The duck bill structures in the Figure 4 and 5 embodiments provide a knotless fixation for the suture. Alternatively, the structures may resist movement of the suture so as to aid the surgeon's ability to maintain tension on the suture while forming a knot.

Although this embodiment depicts the flange of the device resting on the outer cortical surface of the bone, the device may be positioned in a hole which has a counterbore, or countersink, in order to prevent any interference between the flange and other bone or tissues that may come in contact with the site either at rest or during movement. Thus, in one embodiment, the device may be arranged so the device does not extend above adjacent bone surfaces. Even in the case where the device is positioned in a counterbore or countersink feature, the device may contact cortical bone within the countersink or counterbore. Alternately, the device may only contact the outer, cortical surface of the bone, and not extend into a hole in the bone. The device may be held in place by virtue of its engagement with the suture.

Of course, it should be understood that suture fixation devices may be provided in any suitable form. For example, the duck bill portions 45 extending from the flange 43 in the Figures 4 and 5 embodiments may be sized to closely fit into a mating hole formed in bone. This close fit may help in maintaining the suture fixation devices 4 in a desired position in the bone. Alternately, the duck bill structures 45 may be formed so as to be tapered on their outer surfaces. Thus, when the suture fixation device 4 is inserted into a hole in the bone, the tapered surfaces of the duck bill structures 45 may contact the sides of the hole and urge the duck bill structures to move toward each other and lock the suture in place as the suture fixation device 4 is pressed into the hole. In another embodiment, a portion of the suture fixation device 4 that is inserted into a hole may have a screw thread, resilient arms or otherwise be arranged so as to engage the hole and help prevent the suture fixation device from falling from the hole, e.g., before the suture is secured in place. There are many variations of the mechanism form to retain the suture with respect to the device. Some of these forms may require a knot for final fixation. Other capturing mechanisms may provide sufficient locking of the suture such that a knot is not required. Theses are typically known as "knotless" devices. The devices 4 may be made of any suitable material or combination of materials, such as metal, plastic, composites or other. In one embodiment, the suture fixation devices may be made of a bioabsorbable material.

As shown in Figure 6, the suture fixation devices 4 may be handled, e.g., passed through an arthroscopic cannula and set in place with respect to a body portion, using an applier that releasably engages with the suture fixation devices 4. For example, an applier 42 may have a pair of tines 421 that engage with recesses or other features on the suture fixation device 4 so as to removably engage with the suture fixation device 4. The tines 421 may be resilient so that the tines are squeezed together when engaged with the suture fixation device 4. Thus, an elastic force biasing the tine ends apart may help maintain engagement of the tines with the grooves 48 in the suture fixation device 4. Alternately, the tine ends may be force-fit into grooves 48 in the suture fixation device so that engagement is maintained based on friction. Of course, it will be understood that the applier 42 may engage with the suture fixation device 4 in any other suitable way, such as with a screw-in or snap mechanism. As shown in Figure 7, the suture fixation device 4 may be positioned relative to the second opening 54 of the passageway 5 using the applier 42 which may be selectively disengaged from the suture fixation device when the suture fixation device 4 is positioned as desired.

As discussed above, the suture fixation devices 4 has one or more pathways 44. As shown in Figure 8, ends of suture 3 may be passed through respective holes in the suture fixation device 4 using one or more feed members 41. The feed members 41 may have an elongated shape, e.g., a wire or needle shape, that is passed through a respective hole in the suture fixation device 4. A loop at one end of the feed member 41 may receive an end of the suture 3 and thereafter the feed member 41 may be pulled through a respective hole in the suture fixation device 4 so as to pull the suture 3 through the hole. Of course, it should be understood that the suture 3 may be fed through the suture fixation device 4 in any other suitable way. For example, the feed member 41 may include one or more flat plates or strips, e.g., made of metal or plastic, with a hole or recess to accept suture. The flat configuration may allow for easy passage through the restriction portion of the device. Although some resistance may be encountered when feeding the portion of the feed member 41 that engages the suture through the restriction, permanent damage or other compromise of the resistive properties of the restriction may be avoided. When performing this technique, arthroscopically, the suture 3 may be fed through the suture fixation device 4 either inside or outside of the body cavity.

With the suture fixation device 4 in place relative to the second opening 54, the suture 3 may be tensioned so as to appropriately position the rotator cuff 2 relative to the humerus 1. At this point, the suture 3 may be fixed relative to the suture fixation device 4, such as by tying a knot with the suture ends. Thus, the suture fixation device 4 may provide not only a structure to support the suture knot, but also may spread the force of the suture 3 to portions of the relatively hard cortical bone surrounding or otherwise adjacent to the second opening 54. By having the suture fixation device 4 engage with this cortical bone, the suture fixation device 4 may provide a relatively stable and secure fixation point for the suture 3. The suture fixation device 4 may also incorporate a mechanism for knotless fixation of the suture, such as an interference pin, a locking passageway, a locking cap, etc.

Although in the illustrative embodiment described above both ends of the suture 3 are passed through the passageway 5 and secured at or near the second opening 54 of the passageway 5, the suture 3 may be secured in other ways, such as by passing one end of the suture 3 through the passageway 5 and passing another end of the suture 3 around the outside of the bone (e.g., over a portion of the greater tuberosity) where it is secured to the other suture end. In another embodiment, two passageways 5 may be formed through the bone and one end of the suture 3 may be passed through one passageway and the other end of the suture 3 may be passed through the other passageway. The suture ends may then be secured to each other at or near respective second openings of the passageways 5 on the lateral side of the humerus 1. In yet another embodiment, two or more first holes 51 may be formed so as to intersect with one or more second holes 52. Suture 3 may be passed through the two or more first holes 51 and be secured at the second opening 54 of the one or more second holes 52. Such an arrangement may allow for the use of a single second hole 52 and suture fixation device 4 to secure the rotator cuff at two or more points on the humeral head using two or more sutures that pass through different first holes 51. Other suture fixation techniques may be used as desired. However, in all of these techniques, a suture fixation device in accordance with aspects of the invention may be used.

Below, various other aspects that relate to forming a passageway in a body portion, providing suture in a passageway, etc. are described. These aspects may or may not be used with aspects of the invention that relate to a suture fixation device and its use. The aspects of the invention are described below with reference to a surgical procedure regarding the repair of a rotator cuff. However, it should be understood that aspects of the invention may be used in any suitable procedure.

When deciding where to locate the first hole 51 for a passageway 5 in a rotator cuff repair, a surgeon often will wish to first determine the final position for the tissue relative to the bone. To do so, the surgeon may wish to place a suture in the tendon 2 and tension the suture 3 (and thus the tendon 2) so that a desired position for the first hole 51 may be determined, e.g., based on the position of the tendon 2 relative to the bone when under tension.

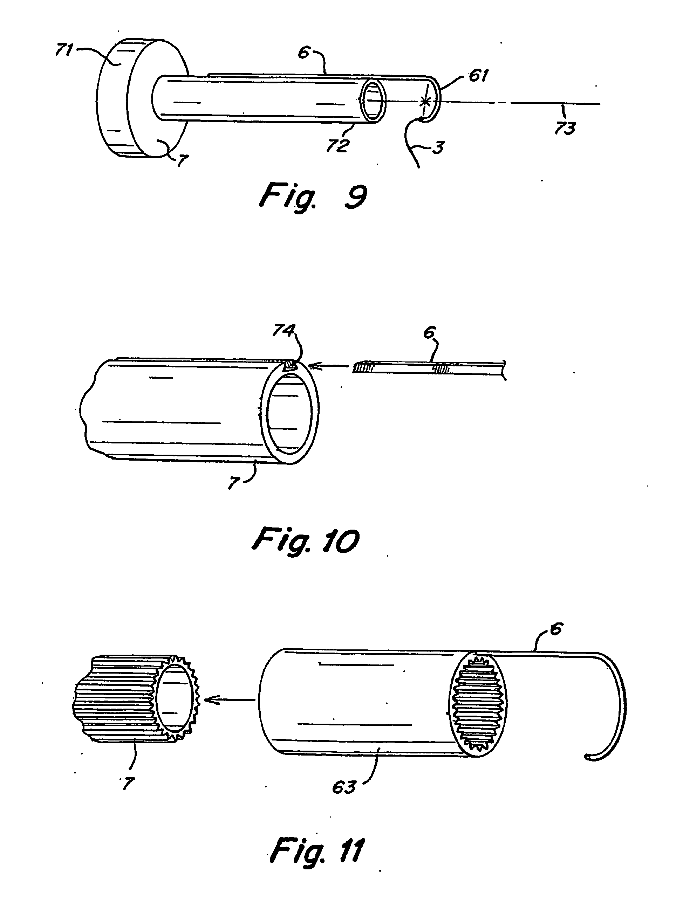

In various aspects, a suture may be placed in the tendon or other tissue 2 using any suitable technique, such as a standard suturing needle and forceps, specialized suturing devices, and so on. However, in one aspect of the invention, use of a needle having a hook-shaped or curved end portion may be preferred. Figures 9-11 show an embodiment of a needle 6 having a hook-shaped tissue penetrating portion 61 at a distal end in accordance with the invention. In the illustrated embodiment, the tissue penetrating portion 61 of the needle 6 has a semi-circular shape and is arranged at an angle, such as 90 degrees to a longitudinal axis of a straight portion 62 of the needle 6. The needle 6 may be formed as a hollow tube so that the suture 3 may pass through the needle 6. Suture may be loaded in the hollow portion of the needle 6 before the surgical procedure is begun, e.g., at the time of manufacture of the needle, or at any suitable time, such as during the surgical procedure. In some cases, the suture may be fed into the hollow portion of the needle 6 before the tissue penetrating portion 61 is formed, e.g., by bending a tube to form a curved end shape.

The arrangement of the needle 6 may allow placement of a mattress stitch in the tissue 2 by rotating the needle as shown in Figures 9-11 so that a tip of the tissue penetrating portion 61 passes through a top side of the tissue 2 and exits from a bottom side of the tissue 2 as shown in Figure 10, and then passes upwardly through the tissue 2 to reemerge at a top side of the tissue 2 as shown in Figure 11. At this point, the suture 3 extending from the tip of the tissue penetrating portion 61 may be grasped, such as by forceps or other gripping device, and the needle 6 may be rotated in reverse so as to again position the needle 6 as shown in Figure 9, thereby leaving the suture 3 positioned in the tissue 2 to form a mattress stitch. During the passage of the suture, the tissue or other material may be held in place, or may be manipulated, by a grasper or other device inserted into the lumen of the cannula. The tissue or other material may also be held in place, or manipulated, by another device, such as a grasper or clamp positioned external to the cannula.

The tissue penetrating portion 61 of the needle 6 may have any suitable shape and may be arranged in a plane that is transverse at any angle to an axis of rotation of the tissue penetrating portion 61 when placing a suture in tissue. That is, although in the illustrated embodiment the tissue penetrating portion 61 has a semi-circular form that lies in a plane at 90 degrees to the rotation axis of the tissue penetrating portion 61 when placing a suture, the tissue penetrating portion 61 need not have a semi-circular form and may lie at any desired angle to the rotation axis. For example, the tissue penetrating portion 61 may be arranged so as to place an inclined mattress stitch in a tissue 2. Further, the needle 6 need not be used only to form a mattress stitch, but rather may be used to form any other suitable stitch type. Also, it is not necessary that the tissue penetrating portion 61 of the needle 6 lie in a single plane. Instead, the tissue penetrating portion 61 may not lie in a single plane, e.g., may have a corkscrew-type or partially helical configuration.

In one aspect, all or portions of a tissue repair procedure may be performed arthroscopically. In this case, and as is known in the art, one or more cannulas may be provided in one or more portals formed in the patient so as to provide access to the operative site. In one aspect of the invention, a needle used to place a suture in a tissue, such as the needle 6 shown in Figure 9, may be used in an arthroscopic procedure. For example, the needle 6 may be secured to a cannula so that the needle may be operated by manipulation of the cannula.

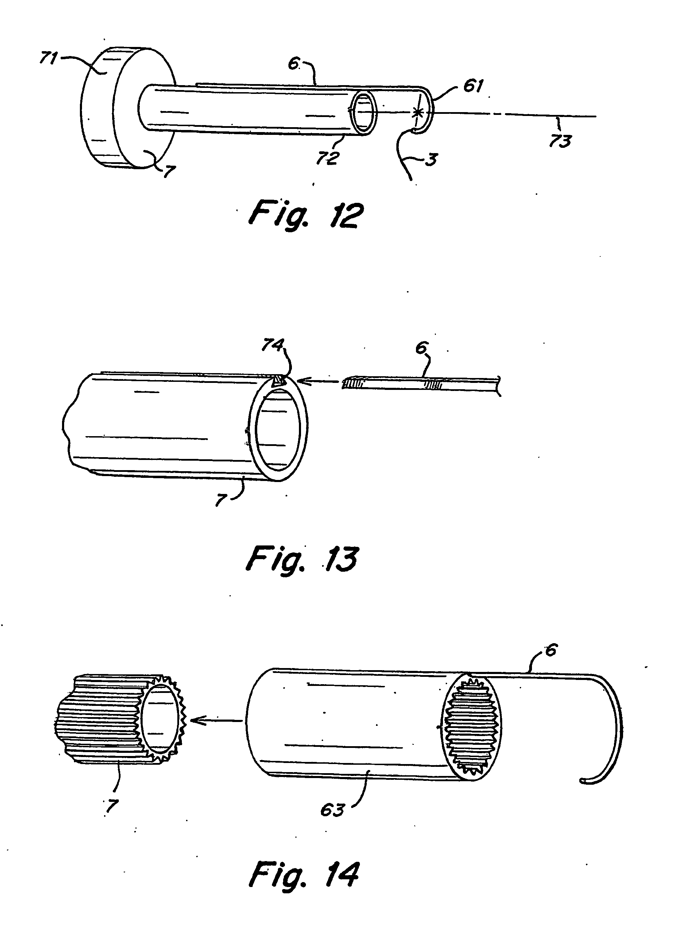

Figure 12 shows an illustrative embodiment of a needle 6 that is secured to a cannula 7. The cannula 7 may have any suitable features found in cannulas used for closed or minimally-invasive surgical techniques, such as one or more valves to resist fluid flow through the cannula 7, an opening through which to introduce a fluid pressure or vacuum, spiral threads or other features on the cannula to aid in placement of the cannula in a portal and/or to help prevent inadvertent removal of the cannula from the portal, and so on. The cannula 7 may be arranged for any type of procedure, such as arthroscopic procedures.

The needle 6 may be secured to the cannula 7 in any suitable way. For example, the needle 6 may be molded into the body of the cannula 7, inserted into the wall of the cannula, may be secured by adhesive, welding, clamps, fasteners, interlocking channels, open channels, or any other suitable device. A proximal end of the needle 6 may terminate at any suitable point, such as midway between a proximal end 71 and a distal end 72 of the cannula 7 as shown, or, more preferably at a position proximal to the proximal end 71. By having the proximal end of the needle 6 positioned proximally of the cannula 7, a user may be better able to access the suture 3 entering the proximal end of the needle 6. The needle 6 may also be axially movable relative to the cannula, e.g., so that the tissue penetrating portion 61 may be moved axially so as to extend away from or toward the distal end 72 of the cannula 7. In addition, although the needle 6 is shown as positioned on an outer surface of the cannula 7, the needle 6, or at least a portion thereof, may be molded into the cannula 7, positioned within the cannula lumen, positioned within the cannula wall, may be arranged within a groove on the outer surface of the cannula, and so on. Although the needle 6 is shown as arranged in an approximately straight fashion along the length of the cannula 7, the needle 6 may be bent, curved or arranged in any suitable way, such as following a spiral path around an outer surface of the cannula 7.

In one illustrative embodiment, a semicircular-shaped tissue penetrating portion 61 of the needle 6 may be arranged relative to the cannula 7 so that a centerpoint of the semicircle lies on a central longitudinal axis 73 of the cannula lumen. Thus, when the cannula 7 is rotated about the central longitudinal axis 73, the tissue penetrating portion 61 may travel in a circular path about the axis 73. However, it should be understood that the tissue penetrating portion 61 may be arranged in any suitable way relative to the axis 73. Further, a plane in which the tissue penetrating portion 61 lies (if present) may be arranged at any angle transverse to the axis 73, and thus need not be arranged at an angle of 90 degrees to the axis 73, as shown in Figure 12.

In one aspect, the needle 6 may be removeably engaged with the cannula 7 so that the needle 6 can be selectively engaged or disengaged with the cannula 7. For example, a cannula 7 may be positioned in a portal in use during a surgical procedure without an attached needle 6. At some point during the procedure, the surgeon may wish to attach a needle 6 to the cannula 7 and manipulate the cannula 7 so as to use the needle 6 to place a suture in a tissue. The needle 6 may be secured to the cannula while the cannula remains in place in the portal (e.g., by inserting the needle 6 into the cannula lumen), or the cannula may be removed from the portal, the needle attached, and the cannula and attached needle inserted into the portal.

Figure 13 shows one illustrative embodiment in which a needle 6 may be removably secured to a cannula 7. In this embodiment, the cannula 7 includes a dovetail-shaped groove 74 into which a correspondingly shaped portion of the needle 6 is inserted. The complementary locking arrangement used by the cannula 7 and the needle 6 need not necessarily be dovetail-shaped as shown in Figure 13, but rather may have any suitable arrangement. Thus, the needle 6 may be selectively secured to the cannula 7 so that rotation or other manipulation of the cannula 7 can cause the needle to be manipulated so as to place a suture in a tissue. The complementary locking arrangement between the needle 6 and the cannula 7 may also allow for axial movement of the needle 6 relative to the cannula 7, e.g., so the tissue penetrating portion 61 can be moved relative to the distal end 72 of the cannula 7.

Figure 14 shows an alternative embodiment in which a needle 6 is fixed to a sleeve member 63 that has one or more complementary locking features that mesh with or otherwise engage with complementary features on the cannula 7. In this embodiment, the complementary locking features have a tooth-like or gear-like form, but the complementary locking features may be arranged in any suitable way. Accordingly, in this embodiment, the needle 6 may be secured to the cannula 7 by sliding the sleeve 63 over the distal end 72 of the cannula 7. It will be understood that rather than having a sleeve 63 that fits over the cannula 7, the sleeve 63 may fit within the internal lumen of the cannula 7, or within a slot in the cannula 7, if desired.

Once a suture is placed in the tissue, such as a rotator cuff tendon, the tissue may be tensioned to determine a location for the opening of the first hole 51 to be formed in the bone. When performing a rotator cuff repair, typically, a first hole 51 of the passageway 5 will be formed vertically from a superolateral position so that the first hole 51 is generally aligned along the length of the humerus 1 and extends into the bone from an opening formed at the margin between the articulating surface 11 and the greater tuberosity 12. This first hole 51 may be formed using a perforator, such as a drill, awl, punch or other suitable device. As with other procedures performed, the first hole 51 may be formed using an arthroscopic portal at a superolateral position, or may be formed in an open surgical procedure.

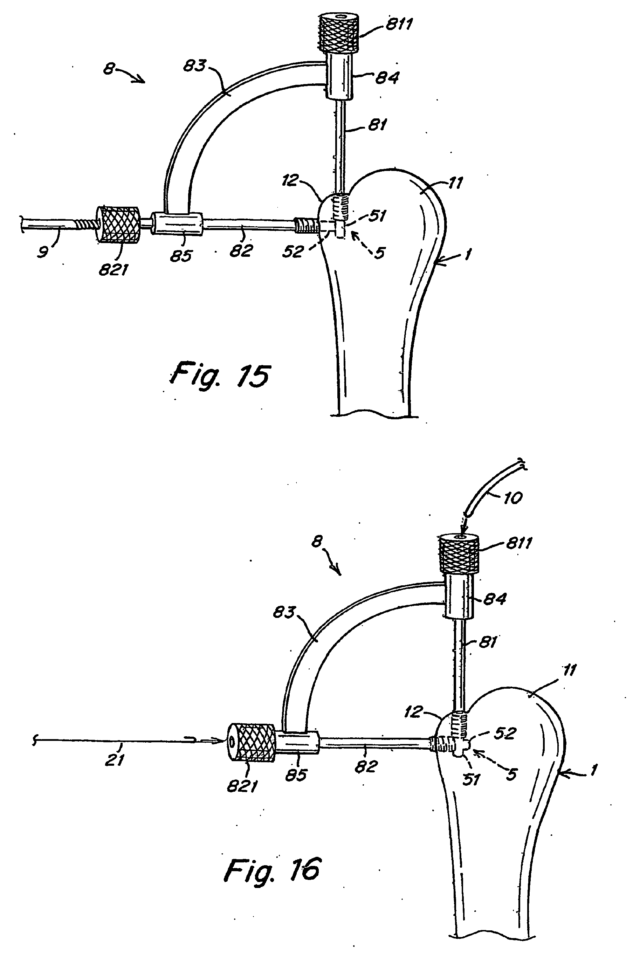

In accordance with an aspect, a guide apparatus may be used to form the first and/or second holes of the passageway (e.g., used to locate a starting point or opening for the first and second holes or used to orient a bone perforator when making the holes), or may be used to help feed a suture or suture-like material through the passageway. For example, a first guide member 81 may be secured relative to the first hole 51, as shown in Figure 15. The first guide member 81 may be part of a guide apparatus 8 used to guide the formation of holes used to form a passageway in bone and/or to pass a suture or other material through the passageway. In the illustrated embodiment, the first hole 51 has been formed in a vertical direction along the length of the humerus 1, e.g., by drilling the hole in a freehand manner. The first guide member 81 may include a feature to help secure the first guide member 81 relative to the first hole 51, such as a threaded distal end that allows the first guide member 81 to be screwed into the bone to a desired depth in the first hole 51. It should be understood, however, that the distal end of the first guide member 81 need not be threaded, but instead may unthreaded and inserted into the first hole 51. Alternately, the distal end of the first guide member 81 may be positioned outside of, but adjacent to, the first hole 51 so that a lumen in the first guide member 81 aligns with the first hole 51. The first hole 51 may be formed so as to be deeper than thought to be needed, e.g., 0.5 cm deeper than a hole depth believed to be required. This overdrilling of the first hole 51 may allow for more flexibility in positioning the first guide member 81 to a desired depth in the bone.

Prior to securing the first guide member 81 relative to the first hole 51, the first guide member 81 may be arranged with respect to a reference structure 83. The reference structure 83 may be used to position first and second guide members 81 and 82 relative to each other in the passageway 5, as is discussed in more detail below. In this illustrative embodiment, the reference structure 83 is arranged so that the first and second guide members 81 and 82 are positioned at a 90 degree angle relative to each other when engaged with the reference structure 83. However, the reference structure 83 may be arranged in any suitable way so as to orient the first and second guide members 81 and 82 at any desired angle relative to each other, including arranging the first and second guide members 81 and 82 in a co-linear fashion. Further, the reference structure 83 may be made so as to be adjustable, thereby allowing the orientation of the first and second guide members 81 and 82 to be changed. For example, the arc-shaped connecting portion of the reference structure 83 may be made so as to be adjustable in length, e.g., having one arc-shaped portion sliding relative to another arc-shaped portion to allow adjustment of the length of the connecting portion. Alternately, or in addition, engagement portions 84 and 85 of the reference structure 83 that engage with the first and second guide members 81 and 82 may be adjustable in orientation relative to the arc-shaped connecting portion. In short, the reference structure 83 may be arranged in any suitable way so as to allow adjustment in the orientation of the guide members 81 and 82.

In this illustrative embodiment, the engagement portions 84 and 85 include sleeves that receive at least a portion of the guide members 81 and 82, e.g., the guide members 81 and 82 may be received in bores in the sleeves. The sleeves may be arranged so that the guide members 81 and 82 are movable linearly along their longitudinal axes and rotationally about their longitudinal axes relative to the engagement portions 84 and 85, but otherwise may be relatively restricted in their range of movement. When a stop on the first guide member 81, such as a knob 811 on the proximal end of the guide member 81, contacts an engagement surface on the reference structure, such as a portion of the engagement portion 84, the second guide member 82 may be positioned by the reference structure 83 so that its longitudinal axis passes a point adjacent the extreme distal end of the first guide member 81. Thus, the second guide member 82 may be used to guide the use of a perforator 9 (such as a drill, punch, awl or other bone perforating device) so that the perforator 9 forms a second hole 52 that intersects with the first hole 51 at a location adjacent the distal end of the first guide member 81. As discussed above, the guide member 82 may guide the movement of the perforator 9, e.g., guide the movement of a drill or punch inserted into a lumen of the guide member 82 as shown, or may guide a starting location for forming the second hole, e.g., be used to mark or otherwise determine a starting location for the perforator 9, but otherwise not interact with the perforator 9.

Alternately, the engagement portion 85 may itself function as a perforator guide with the second guide member 82 being withdrawn from the engagement portion 85. Although in this illustrative embodiment the engagement portions 84 and 85 are shown as relatively short cylindrical sleeves, the engagement portions 84 and 85 may be arranged in any suitable way, e.g., may be elongated so as to more closely approach the humerus 1 and provide improved guidance for a perforator 9 and/or the first and second guide members 81 and 82. Further, the first guide member 81 may be arranged so that is rotationally movable about its longitudinal axis relative to the reference structure 83, but is otherwise held by the engagement portion 84 so that the first guide member 81 is not movable axially. This may aid is appropriately positioning the first guide member 81 and reference structure 83 when forming the second hole 52.

Upon formation of the second hole 52, the second guide member 82 may be screwed into the second hole 52 until a stop on the second guide member 82, such as a knob 821 at a proximal end of the guide member 82, contacts an engagement surface on the engagement portion 85, such as a portion of the sleeve. In this configuration shown in Figure 16 (stops on the first and second guide members 81 and 82 engaged with respective engagement surfaces on the reference structure 83), the extreme distal ends of the first and second guide members 81 and 82 may be adjacent to each other in the passageway 5 formed by the first and second holes 51 and 52. Accordingly, a surgeon may be assured that if the first and second guide members 81 and 82 are positioned within the bone and stops on the guide members 81 and 82 are respectively in contact with appropriate engagement surfaces on the guide apparatus 8, the extreme distal ends of the guide members 81 and 82 will be positioned adjacent each other. Thus, the surgeon may be assured that a wire 10 or other element may be fed into one of the guide members and retrieved from the other of the guide members, e.g., using a retriever 21 having a hook at a distal end. Such an arrangement may be advantageous when using the guide apparatus 8 in an arthroscopic procedure where the operative site may not be easily visualized.

Although in the above embodiment, stops on the first and second guide members 81 and 82 contact corresponding engagement surfaces on the engagement portions 84 and 85, the guide members 81 and 82 may be positioned relative to the reference structure 83 in any suitable way. For example, the guide members 81 and 82 may have indicator marks on them that may be aligned with a portion of the engagement portions 84 and 85, respectively. The alignment of certain indicator marks on the guide members 81 and 82 may be used to indicate, for example, that the distal ends of the guide members 81 and 82 are adjacent each other. Those of skill in the art will understand that the position of the guide members 81 and 82 relative to the reference structure 83 and relative to each other may be determined in other ways.

In this illustrative embodiment, the first guide member 81 is shown as having a smaller diameter (at least at the distal end) than the second guide member 82. This may allow the guide apparatus 8 to be used with an arrangement where the first hole 51 is smaller than the second hole 52. A relatively small first hole 51 may allow for more rapid healing and/or provide additional space for other holes in the margin, if needed. However, it should be understood that the guide apparatus 8 and/or the holes that form the passageway 5 may be made in any suitable way, e.g., the first and second holes 51 and 52 may have the same diameter or the first hole 51 may have a larger diameter than the second hole 52.

Although in this illustrative embodiment, the guide apparatus 8 is used to guide the formation of the second hole 52, the guide apparatus 8 need not necessarily be used to guide the formation of the second hole 52. That is, the guide apparatus 8 may be used only to help feed the wire 10, suture or other material through a passageway that is preformed in the bone or other body portion. In addition, the first and second guide members 81 and 82 may be arranged so that the members 81 and 82 can be secured in a body portion without requiring holes to be predrilled or otherwise formed. Thus, in one embodiment, the first and second guide members 81 and 82 may be arranged like an awl or other device capable of forming a hole in a body portion, e.g., capable of being forced into bone, forming the passageway 5 by their entry and/or providing a means to help feed a wire, suture or other material through the passageway 5.

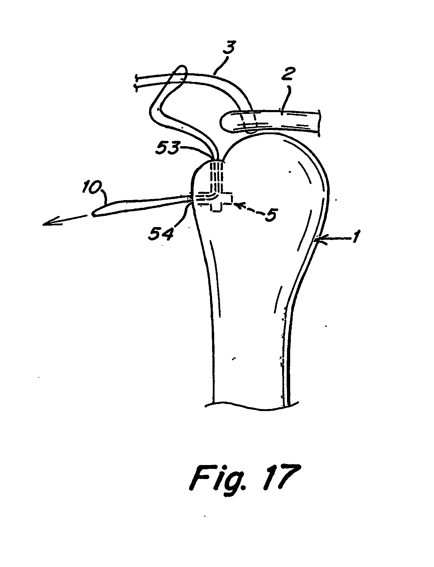

Once the wire 10, suture or other material has been passed through the passageway 5, as shown in Figure 17, the wire 10 may be used to pull the suture 3 through the passageway 5. Prior to being used to pull the suture 3 through the passageway 5, the wire 10 or other material may be used to create a relatively straight pathway for the suture 3 once the suture 3 is tensioned and fixed in place. For example, the wire 10 may be tensioned between the first and second openings 53 and 54 of the first and second holes 51 and 52 or otherwise manipulated so as to cut or crush the body portion, e.g., bone, between the first and second openings 53 and 54. Such manipulation of the wire 10 may perform a kind of "flossing" effect in the bone, allowing the suture 3 to follow a more straight pathway through the passageway 5, reducing the length of suture 3 needed between the rotator cuff 2 and a point of fixation of the suture 3, e.g., near the second opening 54. The wire 10 may have barbs or other saw-like features to aid in cutting bone and forming the pathway. Reducing the length of suture 3 in the passageway 5 may improve the suture's ability to maintain appropriate tension on the rotator cuff 2, e.g., by reducing the amount of stretch of the suture when under tension.

Having thus described several aspects of at least one embodiment of this invention, it is to be appreciated various alterations, modifications, and improvements will readily occur to those skilled in the art. Such alterations, modifications, and improvements are intended to be part of this disclosure, and are intended to be within the scope of the invention. Accordingly, the foregoing description and drawings are by way of example only.

## Claims

1. A suture fixation device for use in a surgical procedure, comprising a body (4) having an inner end and an outer end and a pathway (44) extending between the inner and outer ends, the inner end arranged to be positioned in a hole in tissue and the outer end including a flange portion (43) adapted to be positioned outside of and adjacent the hole, **characterised in that** the inner end of the_body (4) further includes a duckbill structure (45) having two movable portions with opposed surfaces that extend away from the flange portion (43) and are separated by a groove that extends away from the flange portion (43), the movable portions being resiliently biased toward each other to form a restriction in the pathway (44) that relatively freely permits movement of a knotless suture through the pathway (44) in a first direction and inhibits movement of the knotless suture through the pathway (44) in a second direction opposite the first direction.

2. The device of claim 1, wherein the inner end is sized to fit within a hole formed in bone, and the outer end is sized to be larger than the hole and to contact a surface of the bone near the hole.

3. The device of claim 1 or 2, wherein the groove extends across a width of the inner end, and the outer end has a generally flat annular shape.

4. The device of any one of claims 1 to 3, wherein the pathway (44) splits into two sections near the outer end.

5. The device of any one of claims 1 to 4, wherein the restriction provides a knotless fixation of suture relative to the device.

6. The device of any one of claims 1 to 5, comprising a recess (49) in the outer end configured to receive a knot formed in suture extending through the pathway (44).

7. The device of any one of claims 1 to 6, further comprising a knotless fixation mechanism separate from the restriction.

8. The device of claim 7, wherein the knotless fixation mechanism includes a screw mechanism, an interference pin or a locking mechanism.

9. The device of any one of claims 1 to 8, wherein the flange portion (43) is adapted to be positioned outside of and adjacent a suitably sized hole formed in bone, the hole extending from a cortical surface of the bone to cancellous bone, the flange portion (43) being positionable so as to contact cortical bone around the hole; and
the inner end extends from the flange portion (43) and is adapted to be positioned in the hole adjacent cancellous bone.

10. The device of any one of claims 1 to 9, wherein the restriction is arranged to maintain tension on the suture in a direction from the inner end toward the outer end.

11. The device of any one of claims 1 to 10, wherein the device is adapted to secure a suture secured to a portion of rotator cuff and passing through a passageway through bone of a humerus, the passageway extending from a first opening at the margin of the humerus to a second opening at a lateral position on the humerus, where the device is positioned near the second opening.

## Patentansprüche

1. Nahtfadenfixierungsvorrichtung zur Verwendung bei einem chirurgischen Eingriff, die einen Körper (4) mit einem inneren Ende und einem äußeren Ende und einem Pfad (44), der sich zwischen dem inneren und dem äußeren Ende erstreckt, umfasst, wobei das innere Ende dazu eingerichtet ist, in einem Loch in Gewebe positioniert zu werden, und das äußere Ende einen Flanschteil (43) aufweist, der dazu geeignet ist, außerhalb des Lochs und angrenzend an dieses positioniert zu werden, **dadurch gekennzeichnet, dass** das innere Ende des Körpers (4) weiterhin eine Entenschnabelstruktur (45) aufweist, die zwei bewegbare Teile mit gegenüberliegenden Oberflächen hat, die sich von dem Flanschteil (43) weg erstrecken und durch eine Rille getrennt sind, die sich von dem Flanschteil (43) weg erstreckt, wobei die bewegbaren Teile elastisch zueinander vorgespannt sind, um eine Verengung in dem Pfad (44) zu bilden, die eine Bewegung eines knotenfreien Nahtfadens durch den Pfad (44) verhältnismäßig ungehindert in einer ersten Richtung zulässt und eine Bewegung des knotenfreien Nahtfadens durch den Pfad (44) in einer zweiten Richtung, die entgegengesetzt zu der ersten Richtung ist, blockiert.

2. Vorrichtung nach Anspruch 1, wobei das innere Ende so bemessen ist, dass es in ein in Knochen gebildetes Loch passt, und das äußere Ende so bemessen ist, dass es größer als das Loch ist und eine Oberfläche des Knochens in der Nähe des Knochens berührt.

3. Vorrichtung nach Anspruch 1 oder 2, wobei die Rille sich über eine Breite des inneren Endes erstreckt und das äußere Ende eine im Allgemeinen flache Kreisform hat.

4. Vorrichtung nach einem der Ansprüche 1 bis 3, wobei der Pfad (44) sich in der Nähe des äußeren Endes in zwei Abschnitte aufteilt.

5. Vorrichtung nach einem der Ansprüche 1 bis 4, wobei die Verengung eine knotenfreie Fixierung eines Nahtfadens in Bezug auf die Vorrichtung bereitstellt.

6. Vorrichtung nach einem der Ansprüche 1 bis 5, die eine Aussparung (49) in dem äußeren Ende umfasst, die dazu konfiguriert ist, einen Knoten aufzunehmen, der in einem Nahtfaden gebildet wurde, der sich durch den Pfad (44) erstreckt.

7. Vorrichtung nach einem der Ansprüche 1 bis 6, die weiterhin einen Mechanismus zur knotenfreien Fixierung umfasst, der von der Verengung getrennt ist.

8. Vorrichtung nach Anspruch 7, wobei der Mechanismus zur knotenfreien Fixierung einen Schraubmechanismus, einen Eingriffsstift oder einen Arretiermechanismus aufweist.

9. Vorrichtung nach einem der Ansprüche 1 bis 8, wobei der Flanschteil (43) dazu geeignet ist, außerhalb eines geeignet bemessenen, in Knochen gebildeten Lochs und angrenzend an dieses positioniert zu werden, wobei das Loch sich von einer kortikalen Oberfläche des Knochens zu Spongiosa erstreckt, wobei der Flanschteil (43) so positionierbar ist, dass er kortikalen Knochen um das Loche herum berührt; und
wobei das innere Ende sich von dem Flanschteil (43) erstreckt und dazu geeignet ist, in dem Loch angrenzend an Spongiosa positioniert zu werden.

10. Vorrichtung nach einem der Ansprüche 1 bis 9, wobei die Verengung dazu eingerichtet ist, die Spannung auf dem Nahtfaden in einer Richtung von dem inneren Ende zu dem äußeren Ende hin aufrechtzuerhalten.

11. Vorrichtung nach einem der Ansprüche 1 bis 10, wobei die Vorrichtung dazu geeignet ist, einen Nahtfaden zu sichern, der an einem Teil einer Rotatorenmanschette gesichert ist und durch einen Durchgang durch Knochen eines Oberarmknochens hindurchgeht, wobei der Durchgang sich von einer ersten Öffnung am Rand des Oberarmknochens zu einer zweiten Öffnung in einer seitlichen Position auf dem Oberarmknochen erstreckt, wobei die Vorrichtung in der Nähe der zweiten Öffnung positioniert wird.

## Revendications

1. Dispositif de fixation de suture destiné à être utilisé dans une opération chirurgicale, comprenant un corps (4) ayant une extrémité interne et une extrémité externe et une voie de passage (44) s'étendant entre les extrémités interne et externe, l'extrémité interne étant conçue pour être positionnée dans un trou dans un tissu et l'extrémité externe comprenant une partie collerette (43) conçue pour être positionnée à l'extérieur du trou et de façon adjacente à celui-ci, **caractérisé en ce que** l'extrémité interne du corps (4) comprend en outre une structure bec de canard (45) ayant deux parties mobiles présentant des surfaces opposées qui partent de la partie collerette (43) et sont séparées par une gorge qui part de la partie collerette (43), les parties mobiles étant élastiquement disposées en biais l'une en direction de l'autre pour former une restriction dans la voie de passage (44) qui permet relativement librement le mouvement d'une suture sans noeud dans la voie de passage (44) dans un premier sens et empêche le mouvement de la suture sans noeud dans la voie de passage (44) dans un second sens opposé au premier sens.

2. Dispositif selon la revendication 1, dans lequel l'extrémité interne est dimensionnée pour s'ajuster à l'intérieur d'un trou formé dans de l'os et l'extrémité externe est dimensionnée pour être plus grande que le trou et pour venir en contact avec une surface de l'os à proximité du trou.

3. Dispositif selon la revendication 1 ou 2, dans lequel la gorge s'étend sur toute une largeur de l'extrémité interne et l'extrémité externe a une forme annulaire généralement plate.

4. Dispositif selon l'une quelconque des revendications 1 à 3, dans lequel la voie de passage (44) se divise en deux sections à proximité de l'extrémité externe.

5. Dispositif selon l'une quelconque des revendications 1 à 4, dans lequel la restriction assure une fixation sans noeud de suture par rapport au dispositif.

6. Dispositif selon l'une quelconque des revendications 1 à 5, comprenant un creux (49) dans l'extrémité externe conçu pour recevoir un noeud formé dans la suture s'étendant dans la voie de passage (44).

7. Dispositif selon l'une quelconque des revendications 1 à 6, comprenant en outre un mécanisme de fixation sans noeud séparé de la restriction.

8. Dispositif selon la revendication 7, dans lequel le mécanisme de fixation sans noeud comprend un mécanisme à vis, une cheville de serrage ou un mécanisme de blocage.

9. Dispositif selon l'une quelconque des revendications 1 à 8, dans lequel la partie collerette (43) est conçue pour être positionnée à l'extérieur d'un trou dimensionné de façon appropriée formé dans de l'os et de façon adjacente à celui-ci, le trou s'étendant d'une surface corticale de l'os vers de l'os spongieux, la partie collerette (43) étant positionnable afin de venir en contact avec l'os cortical autour du trou ; et
l'extrémité interne s'étend à partir de la partie collerette (43) et est conçue pour être positionnée dans le trou adjacent à de l'os spongieux.

10. Dispositif selon l'une quelconque des revendications 1 à 9, dans lequel la restriction est conçue pour maintenir une tension sur la suture dans un sens allant de l'extrémité interne vers l'extrémité externe.

11. Dispositif selon l'une quelconque des revendications 1 à 10, le dispositif étant conçu pour bien fixer une suture bien fixée sur une partie de coiffe de rotateurs et passant dans une voie de passage dans de l'os d'un humérus, la voie de passage s'étendant d'une première ouverture à la marge de l'humérus vers une seconde ouverture au niveau d'une position latérale sur l'humérus, le dispositif étant positionné à proximité de la seconde ouverture.
